Europäisches Patentamt

European Patent Office

Office européen des brevets

(11) Publication number: **0 091 543**
**B1**

## (12) EUROPEAN PATENT SPECIFICATION

(45) Date of publication of patent specification: **14.06.89**

(21) Application number: **83101471.7**

(22) Date of filing: **18.04.80**

(60) Publication number of the earlier application in accordance with Art. 76 EPC: **0 018 218**

(51) Int. Cl.⁴: **C 07 K 3/00, A 61 K 45/02**

(54) **Anti-interferon antibodies and method of producing same.**

(30) Priority: **20.04.79 DK 1645/79**
**22.02.80 DK 791/80**
**02.04.80 DK 1484/80**

(43) Date of publication of application:
**19.10.83 Bulletin 83/42**

(45) Publication of the grant of the patent:
**14.06.89 Bulletin 89/24**

(84) Designated Contracting States:
**AT BE CH DE FR GB IT LI LU NL SE**

(56) References cited:

**Chemical Abstracts vol. 90, no. 25, 18 June 1979 , Columbus, Ohio, USA G. BODO "Production and purification of human lymphoblastoid interferon", page 454, column 2, abstract no. 202028j & Int. Immunobiol. Symp. (Proc.), vol. 11, 1977, p**

(73) Proprietor: **Technobiotic Ltd.**
**Töpferstrasse 5**
**CH-6004 Lucerne (CH)**

(72) Inventor: **Berg, Kurt Frimann**
**Klintevej 15**
**DK-8240 Risskov (DK)**

(74) Representative: **Walls, Alan James**
**c/o Simmons & Simmons 14 Dominion Street London EC2M 2RJ (GB)**

(56) References cited:
**Chemical Abstracts vol. 90, no. 21, 21 May 1979, Columbus, Ohio, USA S. RUBINSTEIN et al. "Human leukocyte interferon: production, purification to homogeneity, and initial characterization", page 420, column 1, abstract no. 166337y**

Courier Press, Leamington Spa, England.

**Description**

The present invention relates to antibodies raised against or directed substantially only against certain purified forms of human interferon, and to the use thereof in the purification of interferon.

As used herein, the term "protein" includes "glycoprotein".

The Le form of interferon is defined in a paper by E. A. Havell, B. Berman, C. A. Ogburn, K. Berg, K. Paucker, and J. Vilcek, Proc. Nat. Acad. Sci. USA, 72, 2185—2187 (1975).

According to European patent application No. 80301258.2 (the disclosure of which is hereby incorporated by reference), pure human leukocyte interferon proteins have been prepared from crude human leukocyte interferon through a number of special purification steps, and the pure human leukocyte interferon has been characterized by stained protein bands in SDS PAGE (sodium dodecylsulfate polyacrylamide gradient electrophoresis).

More particularly, it was established that under the SDS PAGE and staining conditions described, at a total interferon load of $0.9 \times 10^6$ IFU, ("IFU"=interferon units) pure human leukocyte interferon shows essentially only two sharp stained protein bands at $18,400 \pm 200$ and $20,100 \pm 200$ Daltons, respectively, and a minor stained protein band between $20,300 \pm 200$ and $20,400 \pm 200$ Daltons. As determined by the protein determination described therein, the pure human leukocyte inteferon has a specific activity of about $10^9$ IFU per mg of protein; the specific activity found may vary to some extent depending upon the protein determination method employed, and the specific activity on a protein weight basis was judged to be $2 \times 10^8 - 2 \times 10^9$ IFU per mg of protein. The fact that the pure interferon shows two major distinct bands was in accordance with prior art findings using crude or partially purified interferon preparations which indicated that human leukocyte interferon comprises at least two major species. At a higher total interferon load, e.g. of $3.8 \times 10^6$ IFU, the above-mentioned SDS PAGE system was found to be capable of showing a more differentiated protein pattern comprising six interferon protein bands, i.e. the two strongly stained bands at $18,410 \pm 200$ Daltons and $20,180 \pm 200$ Daltons, respectively, a medium-stained band at $20,420 \pm 200$ Daltons (corresponding to the above-mentioned minor stained band) and just visible protein bands at $19,500 \pm 200$ Daltons, $21,130 \pm 200$ Daltons, and $23,440 \pm 200$ Daltons, respectively. Each of the individual components in the above-mentioned bands of the SDS PAGE acrylamide gradient gel were found to show biological interferon activities: antiviral activity, ability to neutralize only anti-human leukocyte interferon (but *not* anti-human fibroblast interferon), and anticellular activity, plus a variety of so-called non-viral activities, as exmplified by potentiation of Natural Killer cells, potentiation of MLC-CML, increase of HLA antigens, etc.

The complete purification of interferon proteins makes it possible, for the first time, to produce anti-interferon which is strictly specific to the active species described and claimed in European patent application No. 80301258.2 simply by immunizing animals with the pure interferon preparation or one or more of its components. As used herein, the term "monospecific" or "specific" (when applied to the antibodies of the invention) means that the antibodies, when contacted with crude human leukocyte interferon, cross-react to absorb only interferon as claimed in European patent application No. 80301258.2. Such strictly monospecific anti-interferon is extremely useful for antibody affinity chromatography for purification of crude or partially purified *human Le form* interferon to obtain, in a simple and economic way, large amounts of pure *human Le form* interferon or highly purified *human Le form* interferon for clinical purposes, standardization, chemical studies, sequence studies, and as immunogen for repeated preparation of monospecific anti-interferon. It is within the scope of the present invention not only to purify human leukocyte interferon by means of the monospecific antibody raised against the pure human leukocyte interferon, but also to purify other interferon types which cross-react immunologically with the monospecific anti-interferon, e.g. "Namalva" interferon (human lymphoblastoid interferons; the Le form interferon constitutes about 85% of the biological activity of human lymphoblastoid or Namalva interferon, vide E. A. Havell, Y. K. Yip, and J. Vilcek, "Characterization of human lymphoblastoid (Namalva) interferon", J. gen. Virol., 38, 51—59, (1977)) and the interferon containing the Le form obtained by cultivation of a microorganism carrying DNA coding for the production of interferon proteins (or proteins having the significant biological interferon activity determinants).

As it is known that interferon treated with SDS will retain its immunological determinants and even expresses (or preserves) its anti-genicity in a more distinct way compared to non-SDS-treated interferon (as shown by immunizations of mice with human leukocyte interferon preparations of Paucker et al. (Dalton, B. F., Ogburn, C. A., Paucker, K., Production of antibodies to human interferons in mice, Infect. Immun., 19(2), 570—574 (1978), pp 4; 25—30), preparative SDS PAGE makes it possible to not only obtain each of the components in isolated form, but also to perform immunization with the isolated components, such as illustrated in greater detail below.

The antibodies of the invention are, therefore, those raised against or directed substantially only against immunological determinents of human Le form interferon proteins which under the SDS PAGE and staining conditions defined herein at a total interferon load of $0.9 \times 10^6$ IFU show two major sharp stained protein bands having antiviral interferon activity at 18,400 and 20,100 Daltons, respectively, and a minor stained protein band having antiviral interferon activity between 20,300 and 20,400 Daltons, together with smaller peaks of antiviral interferon activity at 19,500, 21,130, and 23,440 Daltons (said Dalton molecular weights being subject to an experimental accuracy of $\pm 200$ Daltons), the stained protein regions of said SDS PAGE acrylamide gradient being essentially only stained interferon proteins; or as human Le form interferon proteins which under the SDS PAGE and staining conditions defined herein at a total interferon load of $3.8 \times 10^6$ IFU show six stained protein bands having antiviral interferon activity, viz. strong bands at 18,410 Daltons and 20,180 Daltons, respectively, a medium-strong band at 20,420 Daltons and just visible bands at 19,500 Daltons, 21,130 Daltons, and 23,440 Daltons, respectively (said Dalton molecular weights being subject to an experimental accuracy of $\pm 200$ Daltons), the peaks of antiviral interferon activity coinciding exactly with the stained protein bands, the stained protein regions of said SDS PAGE acrylamide gradient being essentially only stained interferon proteins.

The invention also relates to antibodies raised against or directed substantially only against immunological determinants of any or a combination of the individual components represented by each of the above-mentioned individual SDS PAGE bands, and of any protein having the significant biological interferon activity determinant(s) possessed by the individual components, and to any protein having the significant immunological determinant(s) possessed by the individual components.

For immunization of animals for the preparation of monospecific anti-interferon of the invention, stabilization of the pure human Le form interferon proteins with SDS (sodium dodecylsulfate) to form an SDS complex of the human Le form interferon protein(s) is a preferred stabilization in view of the fact that SDS increases the antigenicity and/or stability of interferon.

The pH of the pure interferon protein solution for immunization of animals to prepare the monospecific anti-interferon is preferably about 7.2, and a suitable buffer is PBS (phosphate buffered saline).

The stabilized pure interferon protein preparation for immunization of animals may additionally comprise an adjuvant to further increase the antigenicity, and one suitable adjuvant is Freund's adjuvant.

The antigenicity of the pure Le form interferon proteins or each member thereof may be increased and/or stabilized by coupling to an immunogenic carrier (so as to present the pure interferon protein or proteins as a sort of "hapten") in accordance with wellknown principles. As examples of immunogenic carriers may be mentioned PPD (Purified Protein Derivative) and BCG (Bacille Calmette Guérin). However, the use of such immunogenic carriers is not presently preferred.

For immunization purposes, mouse, rabbit, goat and sheep are preferred animals, but it is also within the scope of the invention to use other animals.

In principle, the immunization of animals against the pure interferon is performed in accordance with known methods for preparation of anti-interferon, such as described, for example in Acta Path. Microbiol. Scand. Section B, *83*, 443—460 (1975), but the fact that the interferon proteins of the invention are pure gives rise to minor variations with respect to the concentration of the immunigen and the immunization time and intervals. Examples of immunization schedules appear from the "Experimental Section".

The bleeding of the animal and the isolation of antiserum are performed in accordance with wellknown methods.

The antibodies prepared as described above, are, apart from the trivial fact that they show a natural background characteristic of the animal immunized, substantially specific to the interferon proteins characterized by the above-mentioned SDS PAGE bands. An extremely small amount of impurities not seen as stained bands in the SDS PAGE together with the interferon protein cannot be ruled out. Such proteins which may represent small amounts corresponding to about 1—5% of the total protein content in the pure interferon protein preparation might trigger antibodies against the corresponding impurities. One way of checking such a possibility is to construct an anti-interferon column of the relevant antiserum, obtained by immunizing a rabbit with the pure interferon (that is interferon of the above-described characterization which in SDS PAGE gives the visible interferon protein bands at a load of $1-4 \times 10^6$ IFU in total). The column is constructed without any pre-treating to remove antibodies not directed against interferon. Crude human leukocyte interferon is loaded to the column, and a normal antibody affinity chromatography is performed, vide below. The eluate is analyzed in an SDS PAGE (vide below), and only the interferon bands should then be seen. Possibly faint bands representing up to four other proteins (impurities) may be seen but provided the overall pattern is such that the stained protein regions are overwhelmingly constituted by interferon proteins, i.e. comprise essentially only interferon proteins, then the antibodies are monospecific within the meaning of this specification. This (three proteins) was in fact seen on rabbit anti-serum with a titer of 500,000 IFU-NU/ml in a 2 ml column, loading $2-3 \times 10^6$ IFU of crude human leukocyte interferons.

The "foreign" proteins might also appear by simple spontaneous cross reaction which by chance takes place.

As the stained interferon protein bands in the SDS PAGE have preserved their antigenicity completely or to a considerable extent, it is also possible to use the stained interferon proteins directly cut out from an SDS PAGE as antigen preparations for immunizing immunizable animals such as rabbits. When the stained band cut out from the SDS PAGE is used for the immunization (after preparation described below), a possible cross-over reaction (or contamination from extremely small amounts of impurities) as discussed above is less likely (compared to the total eluate representing 5 interferon species). Thus, antibodies versus the individual species of interferon (primarily the two major species at about 18,400 and 20,100 Daltons) with optimum specificity may be produced according to the following protocol:

1. $4-5 \times 10^6$ IFU human leukocyte interferon (as CIF) is purified completely (by means of the "tandem" affinity chromatography described below) and subjected to SDS PAGE.

2. The gel is only stained for 10—15 minutes at room temperature and is partially destained for 10 minutes followed by a wash in distilled water three times, done in 1—2 minutes with 0°C distilled water. The exact location of the protein bands is noted (for example by means of a Polaroid photo), and the two major interferon protein species are specifically removed by cutting out with a sharp knife. Each slice is minced by means of a teflon rod and 1 ml 0.01% SDS (in PBS, pH 7.2) and is thereafter injected subcutaneously into a rabbit. By following this procedure every second week, low titered antibodies against the human leukocyte interferon proteins are developed in 2—4 months. As soon as a low titer against interferon is detected, Freund's adjuvant is added to the immunogenic mixture every fourth time (every 4th to 6th week) depending on the development of the titers. This procedure is continued for 3—12 months and anti-interferon against the interferon species is developed (10,000—1,000,000 IFU-NU/ml). Thus, the term "monospecific anti-interferon" is used both in relation to anti-interferon produced by means of the pure interferon proteins as described above without the step of cutting out from SDS PAGE, and in relation to the antibodies raised against the stained interferon band or bands cut out from the SDS PAGE.

A further method for producing monospecific antibodies against interferon proteins is the so-called hybridoma technique. The hybridoma technique is a well known method for preparing antibodies and comprises the establishment of monoclonal antibody-producing lymphocytes/myeloma hybrids (compare, for example, "Current topics in Microbiology and Immunology, Vol. 81, Lymphocyte Hybridomas, Eds. F. Melchors, M. Potter, and N. L. Warner, Springer Verlag, 1978). However, until the present invention, it was not known or obvious that it would be possible to obtain an anti-interferon-producing hybridoma cell clone. In the hybridoma technique, using, for example, mouse as the animal immunized, mice are immunized with human Le form interferon and spleen cells from the immunized mice are fused with myeloma cells, whereafter the fused hybridoma are cloned, antibody-producing clones are selected and cultured, and antibodies are obtained from the culturing medium.

Antibodies prepared by hybridoma technique in a mouse system are strictly monospecific and are therefore especially advantageous in radioimmunoassays or other similar tests.

In the hybridoma technique, one particular way of obtaining the antibody is to culture the selected clones in vivo in the animal species from which the spleen cells were derived, and harvesting antibody from the ascites fluid of the animal, and such embodiment is within the scope of the present invention.

The selection of positive hybridoma clones may be performed by the usual interferon neutralization test. However, as the usual interferon neutralization test, as a prerequisite, requires that the antigenic determinant of the interferon is located very close to the center(s) of the biological activity (within a distance of about 1 IgG molecule length), it is likely that antigenic determinants located further away from the center(s) of the biological activity/activities will not be detected by this test, and it is, hence, likely that "positive" hybridoma clones (producing antibodies against antigenic determinants on the interferon protein which are located at a distance from the biological center which is greater than the length of 1 IgG molecule) will escape detection in the test. Therefore, a more advantageous technique for testing for positive hybridoma clones is to use radio-labelled pure human Le interferon proteins of the invention in a radioimmunoassay. The radio-labelled pure human Le interferon proteins can be made by radio-labelling human Le interferon, e.g. a gel filtrate made by the gel filtration technique described below, by means of a standard radio-labelling technique such as using lactoperoxidase and iodine 135, and then purifying the interferon proteins in the manner described herein, subjecting the purified interferon proteins to SDS PAGE and eluting the radio-labelled pur interferon proteins from the SDS PAGE gel. Another method for selecting the positive hybridoma clones in a manner which will detect also such clones that are not detected in the usual interferon neutralization test comprises subjecting an amount, e.g. 500 µl, of the supernatant from each clone cultivation to immobilization on a matrix, e.g. immobilization on CNBr-activated Sepharose according to the method described in the section "Materials and Methods", applying human Le form interferon, e.g. crude human leukocyte interferon, to the resulting treated matrix, e.g. by mixing the resulting matrix gel suspension corresponding to each clone with the interferon and allowing the mixture to stand for a period, e.g. 1 hour at 37°C, effectively separating unbound interferon from the matrix material, e.g. by centrifugation and washing with PBS, and thereafter subjecting each matrix gel portion to elution to release any bound interferon, e.g. by mixing with elution buffer (pH 2.4) and centrifugating, and selecting the clones corresponding to the matrix gel portions from which the eluting buffer portions, in particular the last eluting buffer portions, contain interferon, as the yielding of interferon in the elution is an indication of a positive clone. The two above-mentioned advantageous methods for detecting positive hybridoma clones may be applied not only to anti-interferon-producing hybridoma clones, but with evident modifications, also to the detection of positive hybridoma clones producing antibody directed against other proteins.

Interestingly, it has been found that antibodies raised against one of the purified interferon proteins of the invention are capable of neutralizing the other purified proteins of the invention. Thus, as will become apparent, the monospecific antibodies of the invention, whether raised against a single purified interferon protein of the invention or raised against a combination of purified interferon proteins of the invention, are equally effective for purification of human Le form interferon-containing solutions.

In accordance with wellknown principles, the monospecific anti-interferon of the invention can be used for determination of the corresponding interferon or interferon component in biological fluids such as by radioimmunoassay or related techniques. However, as alluded to above, an interesting and important

EP 0 091 543 B1

utility of the monospecific antibodies is for antibody affinity chromatography purification of interferon-containing solutions. For this purpose, the antibodies are immobilized on a matrix in a manner known *per se*, suitably covalently bound to a suitable antibody affinity chromatography matrix such as a cross-linked agarose such as Sepharose 4B from Pharmacia. The antibody affinity chromatography purification of interferon-containing solutions may be performed according to any of the wellknown methods, either batchwise or, preferably, using the matrix-immobilized antibody arranged in a column.

The preparation of antibody affinity columns using the monospecific anti-interferon, and the operation of such columns are performed in a manner known *per se*. The interferon-containing solution applied on such columns may be a crude, unconcentrated interferon preparation, or it may be a concentrated or partially purified interferon preparation. The interferon preparation applied on the column may be any interferon preparation containing human Le form interferon, that is, human leukocyte interferons, human lymphoblastoid interferons (Namalva interferons), or interferon (or important parts thereof) produced by cultivation of a microorganism containing DNA coding for interferon, such as described above. The use of antibodies against partially purified human leukocyte interferon in antibody affinity chromatography for purifying Namalva interferon and leukocyte interferon has already been described (vide, e.g. Scand. J. Immunol. *8*, 429—436 (1978)). However, the important improvement is that monospecific anti-interferon will retain substantially only human Le form interferon protein, the remaining proteins of the preparation passing through the column. Very small amounts of impurities due to spontaneous cross-reactivity cannot be ruled out, not even when the antibodies used are antibodies produced by hybridoma technique which must, apart from this, be expected to "produce" (react with) only pure interferon proteins.

At suitable dimensions of such antibody columns (which can be designed in accordance with wellknown principles for antibody affinity chromatography columns), the columns may be used for large scale industrial purification of interferon from a crude interferon preparation to result in pure (or highly purified) interferon proteins in the column eluate. The pure (or highly purified) interferon proteins prepared in this way are stabilized with suitable stabilizers according to the intended use thereof, such as described above.

As the interferon of the interferon preparations applied on the monospecific anti-interferon columns is present in usually very low concentrations, on a weight basis, and as as great amounts as possible of the valuable interferon are to be isolated, it is of importance to minimize any deterioration of the interferon proteins which might be caused due to the presence of proteolytic activity in any biological subtance with which the interferon is contacted, and one aspect of the present invention comprises removing any proteolytic activity from any biological material with which the interferon to be purified is in contact.

One important utility of this aspect is the removal of proteolytic activity from the anti-interferon antibodies (immunoglobulins) of the invention. According to the invention, this removal is suitably performed by treating the antibodies, prior to their binding to the matrix, with matrix-immobilized enzyme inhibitor or enzyme destructure which is not harmful to immunoglobulins (or the important fragments thereof). Thus, the antibodies may be passed through a column of matrix-immobilized poly-L-lysine and/or matrix-immobilized Soyabean Trypsin inhibitor, and/or matrix-immobilized kallikrein inactivator. An example of a suitable treatment of the antibodies is passage through a column of poly-L-lysine covalently bound to cross-linked agarose such as Sepharose 4B, followed by passages through a column of Soyabean Trypsin inhibitor covalently bound to the same matrix. It has been found that this removal of proteolytic activity increases the recovery of interferon activity in antibody affinity chromatography purification of interferon-containing solutions.

The monospecific anti-interferon, when covalently bound to a matrix such as cross-linked agarose, is preferably bound to such an extent that the total amount of antibody covalently bound to the matrix corresponds to at the most 85% of the immunoglobulins used at the covalent binding stage, such as described by the present inventor in Scand. J. Immunolog., *6*, 77—86 (1977). This results in the highest recovery of interferon from the column.

When the eluate from the monospecific anti-interferon affinity chromatography column is to be used for administration in human beings, it is important that it does not contain any components which might be immunogenic in man. One risk which might be associated with antibody affinity chromatography is that immunoglobulins or immunoglobulin fragments liberate from the column and become eluted together with the desired protein or proteins.

According to the invention, such immunoglobulins or fragments thereof which are immunogenic in man are removed by passage of the eluate through an antibody affinity column in which the antibodies are directed against the anti-interferon immunoglobulins and are of a kind which is non-immunogenic on parenteral administration to human beings. (Prior to the passage of the eluate through the said column, it should be adjusted to a neutral pH, e.g. by dialysis against PBS, pH 7.2).

The purification of human Le form interferon proteins is described in European patent application No. 80301258.2.

Materials and methods

*Interferon assays* were performed according to the well-known standard method (Berg K., Sequential Antibody Affinity Chromatography of Human Leukocyte Interferon, Scand. J. Immunol. *6*, 77—86 (1977)) using VERO cells (monkey kidney cells) and Vesicular Stomatitis Virus (VSV) as a challenge virus. All

5

interferon units (IFU) are expressed in international reference units (69/19 B units) (69/19 B reference was obtained from MRC, Mill Hill, U.K.)

Interferon.

Crude human leukocyte interferon was produced according to the method as described by Cantell (Cantell, K., Hirvonen, S., Mogensen, K. E. and Pyhälä, L., Human leukocyte interferon: production, purification, stability and animal experiments. In: The Production and use of Interferon for the Treatment and Prevention of Human Virus Infections pp. 35—38, Waymouth, C. (ed.). Proceedings of a Tissue Culture Association Workshop held at Lake Placid, 1973 (In Vitro Monograph, volume 3), Tissue Culture Association, Rockville, Md.) using Sendai virus as interferon inducer. Partially purified interferon (PIF) with a specific activity of $5 \times 10^5$ IFU/mg protein was obtained from crude concentrated human leukocyte interferon (CIF) by ethanolic precipitation as described by Cantell, K., Hirvonen, S., Mogensen, K. E. and Pyhälä, L., loc. cit.

Crude Namalva interferon was produced substantially as described by Strander et al., Production of human lymphoblastoid interferon, J. Clin. Microbiol. *1*, 116—124 (1975), using Sendai virus as interferon inducer.

*Interferon neutralization* for determining anti-interferon was performed in a micro-assay system in the following manner: 20,000 VERO cells per well were seeded in 100 μl medium and kept at 5% $CO_2$ in a humidified cabinet. On day 2 the medium was removed from the cells, and each well received 100 μl of a dilution (in medium) of the antiserum, containing an interferon concentration of 6—8 IFU/ml (the serum and interferon had been preincubated at 37°C for 1 h). On day 3 the medium was removed, and all the wells received 100 μl VSV (diluted to $10^{-3.5}$ in medium). On day 4 the CPE (cytopathogenic effect) was determined, and 50% destruction was taken as the end point for the determination of the anti-interferon titer. The titers are expressed as interferon neutralization units (IFU-NU) per ml.

*Non-monospecific anti-interferon* against PIF was produced, according to Mogensen, K. E., Pyhälä, Liisa and Cantel, K., Acta path. microbiol. scand. Sect. B, *83*, 443—450, (1975), partly in sheep, partly in rabbits. The titer of the sheep anti-interferon was 100—250,000 IFU-NU/ml. For the preparation of the rabbit anti-interferon, a rabbit was injected weekly, s.c. with PIF ($2 \times 10^5$ IFU) for more than two years. The titer of the rabbit anti-interferon was 15,000—30,000 IFU-NU/ml. All immunoglobulins were isolated by 50% ammonium sulphate precipitation, followed by a dialysis versus phosphate buffer saline (PBS), pH 7.2.

Chemicals

CNBr was from Fluka (stored at −20°C). Sodium dodecylsulphate (SDS), specially pure for electrophorese, was purchased through British Drug House (BDH). Soyabean Trypsin Inhibitor (STI) and L-Lysine were obtained from Sigma. Sepharose 4B, CNBr-activated Sepharose 4B, CH-activated Sepharose 4B, and Epoxy-activated Sepharose 6B were all purchsed from Pharmacia (Denmark).

Binding procedures

The covalent binding of the immunoglobulins to Sepharose 4B was done as previously described by K. Berg in Scand. J. Immunolog., *6*, 77—86, (1977). Only 80—85% of the immunoglobulins were deliberately bound.

*Protein determinations* were made by a modification of the Lowry procedure (Berg K., Sequential Antibody Affinity Chromatography of Human Leukocyte Interferon, Scand. J. Immunol., *6*, 77—86 (1977)) which permitted detection of 1—2 μg/ml as the lowest level of proteins detectable (using an LKB Calculation Absorptioner Ultralab System). Crystalline bovine serum albumin was used as a standard protein. To determine the protein concentration of the purified interferon (1—5 μg in total) the following procedure was adopted: SDS was added to a final concentration of 0.1%. The lyophilized protein sample was further examined on an SDS-polyacrylamide gel electrophorese (SDS PAGE, see later), subsequent to a dialysis versus distilled water. The intensity of the stained protein bands was compared with known standards in different amounts (see later, under SDS PAGE), and the total amounts of proteins were estimated The deviations were 5—10%, with the lowest detectable level of proteins being 0.1 μg (in total). The results from this method will serve as a rough estimate, rather than as an actual measurement.

*Affinity chromatographies* were performed at 4°C. The gel suspensions were degassed before packed into the columns. Packing was performed by washing with 3—5 bed volumes of loading buffer, using a peristaltic pump. Samples (100 μl) for interferon titrations were taken from either pools or individual fractions and titrated on the same day or frozen in plastic tubes (−20°C) and titrated later. The dilutions were made in medium (incl. 10% calf serum).

*Antibody affinity chromatography* was essentially done as described by Berg (Sequential Antibody Affinity Chromatography of Human Leukocyte Interferon. Scand. J. Immunol., *6*, 77—86 (1977)). As loading buffer was used 0.1 M NaOA/0.3 M NaCl at pH 7.2 (flow rate 40 ml/h). Stepwise elution was performed with 0.1 M HOAc/0.3 M NaCl including a minute amount of citric acid (enough to keep the pH firmly at 2.4). When not operated, the column was stored at 4°C in PBS 1 M NaCl including Penicillin, Streptomycin, Gentamycin and Chloramphenicol (1% of each). Before using the column for purification purposes, it was first washed with 100 ml of loading buffer followed by 10 ml of eluting buffer and finally equilibrated with 20—30 ml of loading buffer. This washing-cycle was necessary to avoid "spontaneous" proteins, especially when working with interferon of specific activities above $10^7$ IFU/mg proteins. The plastic tubes used for collecting the interferon eluate were pre-wetted with 100 μl of 1% SDS.

SDS PAGE.

The purified, concentrated interferon preparations were analyzed for polypeptides components on SDS PAGE slab gels using 20 cm long separating gels, 0.75 mm thick (Bio Rad model 221: Dual vertical slab gel electrophoresis cell) and 7—10 cm long stacking gel. Exponential gradient gels of about 9—22% polyacrylamide were prepared by mixing 11 ml 22% acrylamide solution with about 32 ml 9% solution in a simple, ice-cooled gradient-device, as described in Knight, E., Interferon: Purification and initial characterization from human diploid cells. Proc. natn. Acad. Sci. USA 73, 520—523 (1976). The discontinuous buffer system, as described by Laenomli (Laenomli, U.K., Cleavage of Structural Proteins During Assembly of the Head of Bacteriophage T4, Nature 227, 680—685 (1970)) was used. The gel was pre-cooled for 2 h (10°C) before starting the actual electrophorese which was performed overnight (10°C) at constant effect (LKB power supply), starting out with 10 mA (and about 20 V).

Samples to be analyzed were dissolved (or diluted) in 0.1 M Tris, HCl (pH 6.8) 2.5% SDS and 5% glucose including a tracking dye (sample buffer). The gel was stained in Comassie Blue (1.25 mg/ml in 50% methanol, 40% $H_2O$ and 10% acetic acid), without prior fixation, for 15 minutes at room temperature under constant rocking, and destained in 7% acetic acid (5% methanol). The gels were dried on paper of a good quality (for example, Whatman Chromatographic paper (17 mm)) under heat and vacuum using a gel dryer (Bio Rad, gel slab dryer, model 224). Solutions of five different molecular markers, from 0.1 μg to 10 μg of each marker per 20 μl,—Lactalbumin (14,400 Daltons); Soyabean Trypsin Inhibitor (20,100 Daltons); Carbonic Anhydrase (30,000 Daltons); Ovalbumin (43,000 Daltons); Bovine Serum Albumin (67,000 Daltons); Phosphorylase (94,000 Daltons) (obtained as an electrophoresis calibration kit (Pharmacia, Denmark))—were subjected to SDS PAGE and stained. It should be noted that molecular weights assessed in this manner are subject to experimental accuracy of about ±200 Daltons. The stained protein bands were compared with the corresponding bands obtained from a parallel SDS PAGE of a purified interferon preparation and the total concentration of interferon proteins was estimated. For obtaining a biological profile from an SDS PAGE, the part of the gel intended for interferon determination, was cut from the remainder gel and kept at 4°C (in a humidified box) on a glass plate. The main part of the gel was stained for 15 minutes; after destaining for additionally 3—5 minutes, weak bands were clearly seen on a blue background, whereby the precise location of the protein bands corresponding to 14,000 and 30,000 Daltons could be established. The unstained part of the gel was cut, so it only contained proteins between 14,000 and 30,000 Daltons and was further subdivided in 1 mm pieces by sharp knives. The interferon from these slices are eluted with 0.5 ml 0.1 M SDS subsequent to a complete mincing by means of a teflon rod. After 5 h at room temperature (rocking) the interferon activity of the supernatant was determined. The individual fractions were frozen at −20°C without any additives.

Experimental section
Preparation of pure human leukocyte and lymphoblastoid interferon proteins

Concentration of crude human keukocyte interferon.

To 3 liters of crude human leucocyt interferon was added KSCN up to a concentration of 0.5 M at pH 7.2. The pH was lowered by addition of 1N HCl to 4.5 (magnetic stirring) whereby a protein precipitate containing the interferon (and part of the impurities) was obtained. The precipitate was dissolved in 150 ml of PBS (phosphate buffered saline, pH 7.2) including 1 M NaCl and 25% by volume of ethylene glycol and dialyzed thoroughly versus 3 times 2 liters of the same buffer at 4°C. The specific activity of the crude concentrated human leukocyte interferon (HuLeCIF) was 5—10×$10^3$ IFU/mg protein. The recovery was about 98%.

Concentration of crude Namalva interferon

To 1 liter of crude Namalva interferon, with a titer of about 8000 IFU/ml, was added KSCN up to a concentration of 0.5 M at pH 7.2. The pH was lowered by addition of 1N HCl to 4.5 (magnetic stirring) whereby a protein precipitate containing the interferon (and part of the impurities) was obtained. The precipitate was dissolved in 50 ml of PBS, pH 7.2, including 1 M NaCl and 25% by volume of ethylene glycol and dialyzed thoroughly versus 3 times 2 liters of the same buffer at 4°C. The specific activity of the crude concentrated Namalva interferon (NaCIF) was 10—12×$10^3$ IFU/mg protein. The recovery was about 98%.

Gel filtration

A 100 cm long column (2.6 cm in diameter, Pharmacia K 2.6/100) was packed with Ultrogel AcA 5/4 (LKB Denmark) in PBS containing 1 M NaCl and 25% by volume of ethylene glycol at 4°C (pH 7.2). After washing the column with 3 bed volumes of buffer, the column was stabilized. 10—15 ml of HuLeCIF (prepared as described above in 25% by volume of ethylene glycol, 1 M NaCl in PBS, pH 7.4) were loaded to the column, and the column was "eluted" with the loading buffer, the fractions being assayed for interferon activity. The interferon-containing fractions were pooled, and about 95% of the original interferon activity was recovered. The specific activity of the gelfiltered human leukocyte interferon-containing eluates was

7

close to 1,000,000 IFU/mg protein, corresponding to a purification factor of 200. As determined by means of molecular markers, the molecular weight of the interferon corresponds to a range of 10,000—20,000 Daltons. Titrations of individual fraction revealed only one broad peak, with a maximum at 18,000 Daltons.

In the same manner as described above, 10 ml of NaClF (prepared as described above in 25% by volume of ethylene glycol, 1M NaCl in PBS, pH 7.4) were loaded to the column, and the "elution" was performed in the same manner as described above. The recovery was about 90%. The specific activity of the gelfiltered Namalva interferon-containing eluate was close to 1,000,000 IFU/mg protein, corresponding to a purification factor of 100. As determined by means of molecular markers, the molecular weight of the interferon corresponds to a range of 10,000—20,000 Daltons. Titrations of the individual fractions revealed a broad peak, with a maximum at 18,000 Daltons.

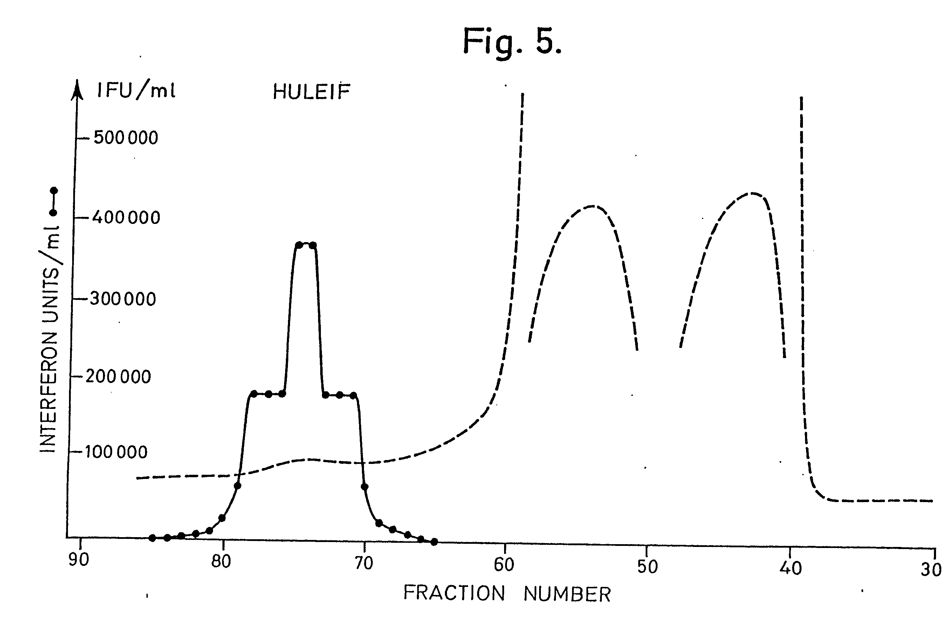

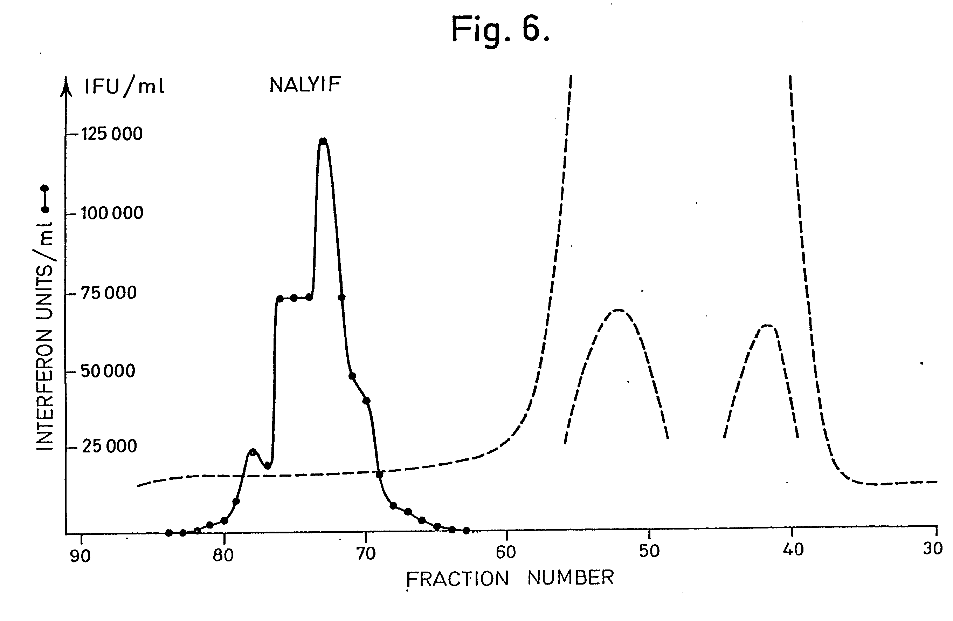

The gel filtration curves for the above-described gel filtration of HuLeCIF and NaCIF are shown in Figures 5 and 6, respectively, and "HULEIF" indicates the human leukocyte interferon, whereas "NALYIF" indicates the Namalva (lymphoblastoid) interferon. It is clearly seen that the interferon activity is effectively separated from the major part of the proteins.

Blue dextran chromatgraphy

The gel-filtered human leukocyte interferon solution, obtained as described above, was exhaustively dialyzed against 200 volumes of 20 mM PB, pH 7.2 at 4°C. The dialysis was performed twice, the total dialysis time being about 24 hours. The dialyzed solution (25 ml, containing $1.8 \times 10^6$ IFU) was loaded on a column of Blue Dextran-Sepharose 4B. The diameter of the column was 1 cm, and the length of the column was 10 cm. The column was pre-washed with 200—300 ml of 20 mM PB (phosphate buffer) at pH 7.4. The dialyzed interferon preparation was loaded to the equilibrated column, and the column was washed with 75 ml of PB. 4500 IFU was found in the wash. The column was eluted with 0.6 M NaCl, 20 mM PB, pH 7.2 whereby more than 95% of the interferon activity was recovered in 6 ml of eluate, as determined by interferon titration.

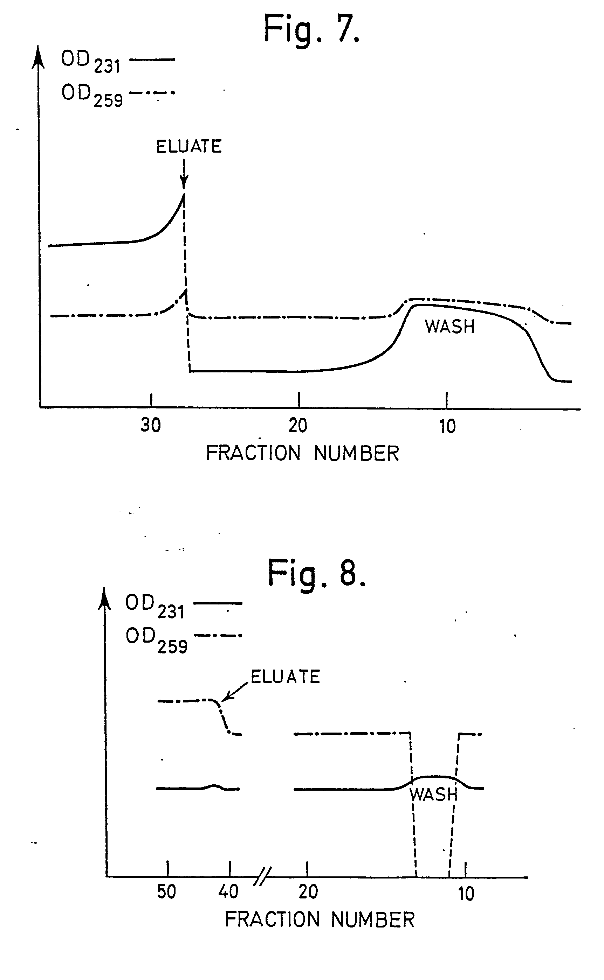

In exactly the same manner, the above-mentioned gel-filtered Namalva interferon solution was exhaustively dialyzed and thereafter subjected to Blue Dextran chromatography. The input in the Blue Dextran chromatography was 1,600,000 IFU. The wash consisted of 70,000 IFU in 50 ml. The eluate was obtained by means of 0.6 M NaCl in PB (pH 7.2). The Blue Dextran chromatography of Namalva interferon is illustrated in Figure 7. The fibroblast part of the Namalva interferon was not eluted from the column under the above conditions, but is expected to be eluted using, e.g., 25% ethylene glycol in 1 M NaCl, pH 7.2.

The above-mentioned Blue Dextran column was a column of Blue Dextran (Cibacron Blue F3GA immobilized on Dextran 2000 (molecular weight 2 millions)) coupled to cyanogen bromide-activated agarose (Sepharose 4B). Thus, the more complete designation of the column is Blue Dextran-Sepharose 4B. This type of column is described by Bollin et al., *loc. cit.* After elution, the column was cleaned by elution with 25—30 ml 25% ethylene glycol, 1.5 M NaCl in 20 mM PB. The column was stored in this buffer at 4°C when not in use. As mentioned above, the loading conditions could also involve the use of hydrophobic reagents, such as alcohols in various amounts (0—50%).

The 0.6 M NaCl eluates from the Blue Dextran chromatography show a specific activity of $70 \times 10^6$ IFU/mg of protein, both for the human leukocyte interferon and for the Namalva interferon. Thus, these are candidates for parenteral administration in human beings for therapeutic purposes and, in this regard, are much more pure preparations than the commonly used PIF preparations. For this use, the eluates are stabilized with physiologically acceptable stabilizers such as described further above, for example 1% of human albumin.

For further purification and for preparation of pure interferon, the eluates from the Blue Dextran column are directly transferred to an antibody affinity chromatography column. In the most advantageous embodiment, the antibody affinity chromatography column is combined with the Blue Dextran column in a "tandem system" as described below:

Tandem affinity chromatography

Instead of eluting the Blue Dextran column as described above, the Blue Dextran column is combined with the equilibrated antibody column prior to the elution, by connecting the outlet of the Blue Dextran column with the inlet of the antibody column. In this manner, the eluate from the Blue Dextran column is immediately "caught" by the antibody column. This combination makes use of the fact that the elution conditions (0.6 M NaCl, 20 mM PB, pH 7.2) can be used as loading conditions of the antibody column. After the elution/loading using 20 ml of the eluate/"loading buffer" (this "loading buffer", of course, at the same time contains the interferon eluted from the Blue Dextran column), the two columns are disconnected, and the antibody column is washed further before eluted as described above. The human leukocyte interferon eluate from the antibody column contains pure interferon proteins showing a specific activity of more than $10^9$ IFU/mg of protein (as assessed by the determination method discussed above). For stabilization of the pure interferon proteins, the tubes in which the eluate from the antibody column is collected (fraction size 2 ml) have been pre-wetted with 100 µl of 1% SDS each. After pooling of the interferon-containing eluate, additional SDS is added up to a total concentration of 0.1% by weight.

The pooled interferon-containing eluates stabilized with 0.1% SDS are transferred to a 20 ml stainless steel tube pre-cooled to 0°C in an ice bath. After 15 minutes, a precipitate is formed. The precipitate is isolated by centrifugation at 20,000 rpm at 4°C for 20 minutes. The supernatant is discarded (no interferon activity), and the precipitate is redissolved in 4 ml of 8 M urea and transferred to a Millipore concentration cell, size 8 ml, filter 10,000 molecular weight cut, and concentrated to about 100 µl at room temperature. Thereafter, additional 4 ml 4 M urea (p.a.) was added to the concentrate, and the solution was concentrated to about 100 µl at room temperature. 1—3 ml of distilled water was added, and the solution was concentrated again to a volume of 20 µl and mixed with 20 µl SDS sample electrophoresis buffer. 20 µl of the resulting solution was used for characterization as described in the section "SDS PAGE" below.

The above-mentioned antibody affinity chromatography column had been prepared in accordance with "Binding Procedures" using non-monospecific anti-PIF which had been absorbed as follows: a total amount of $10^6$ IFU-NU of anti-interferon immunoglobulins (corresponding to 4 ml sheep anti-interferon serum) was absorbed three times on a 150 ml column of human serum bound to Sepharose 4B followed by 4 absorptions on a CIF-epoxy Sepharose column and 2 absorptions on a CIF CH-activated Sepharose 4B as described in the below section "Absorption of Anti interferon" and in Scand. J. Immunol. 8, 429—436 (1978). Finally, the immunoglobulins had been absorbed on a poly-L-lysine-Sepharose column (once) and on a Soyabean Trypsin Inhibitor-Sepharose column (twice).

The eluate from the Blue Dextran chromatography of Namalva interferon was divided in two portions. One portion was used for SDS PAGE electrophoresis as described below. 250,000 IFU were loaded to the absorbed antibody column as shown in Figure 8. No interferon was found in the wash. The interferon was eluted as usual by lowering pH to 2.4, and 235,000 IFU (collected in the presence of 0.1% SDS) were recovered. This eluate was concentrated as described above and further examined in SDS PAGE.

SDS PAGE.

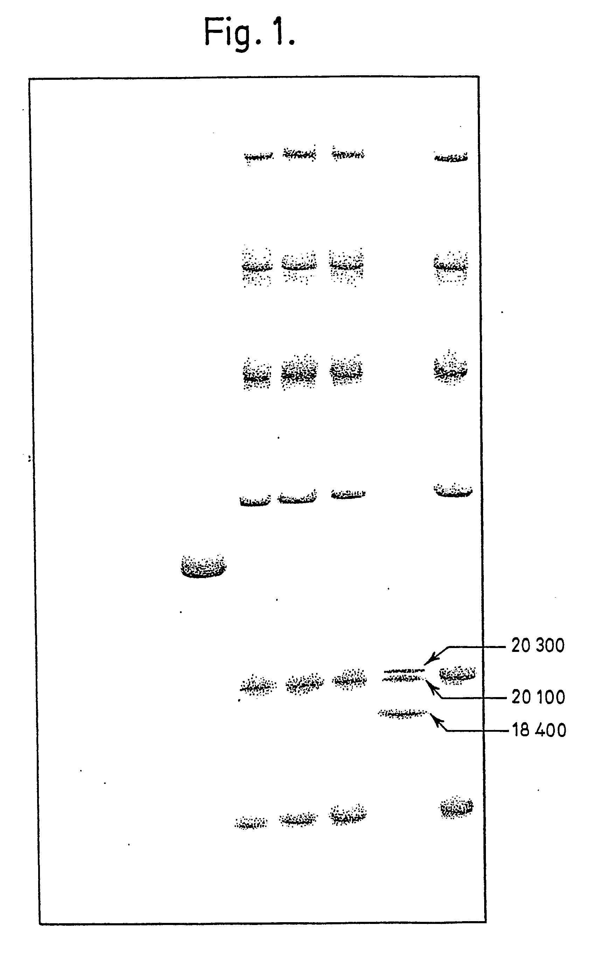

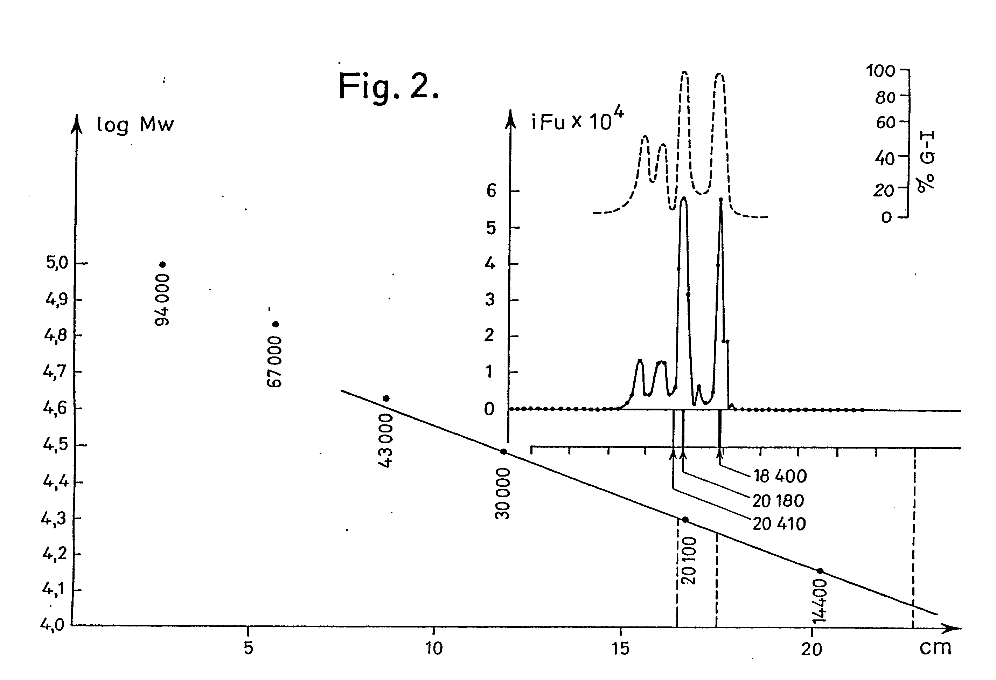

The SDS PAGE electrophoresis was performed as described under "Materials and Methods" above. The stained slab of the electrophoresis of the pure human leukocyte interferon proteins is shown in Figure 1. Figure 2 shows, schematically, the stained slab from another experiment, together with the corresponding interferon activity eluted from an unstained parallel gel strip. The striking reproducibility between the two experiments appears from the two Figures, the difference between 20,100 and 20,180 being within the experimental accuracy. As mentioned previously, the biological peaks coincide exactly with the proteins.

From Figure 1, it appears that the interferon preparation is completely pure by SDS PAGE. There is no other protein band whatsover visible.

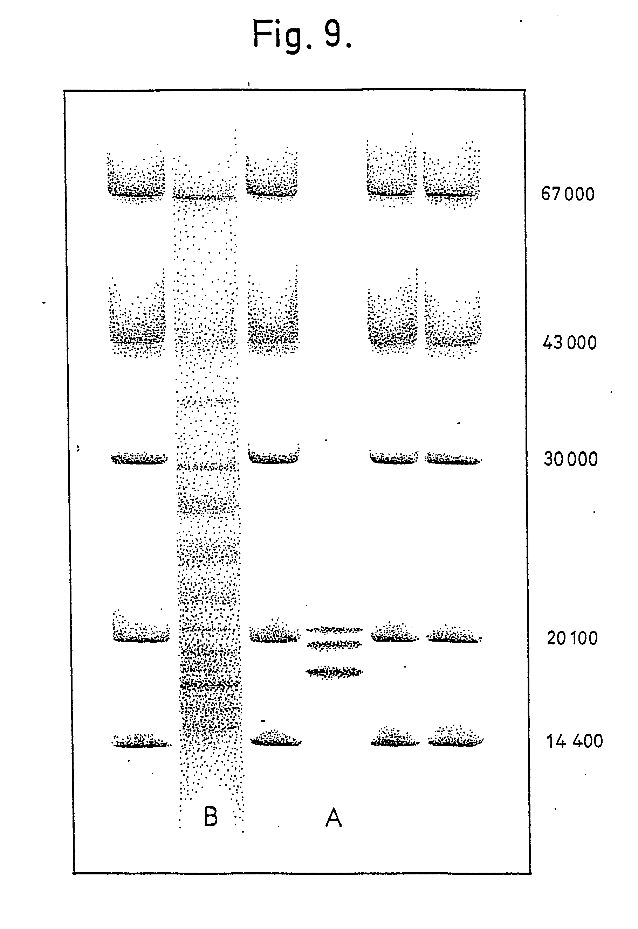

Figure 9 shows the stained slab from the SDS PAGE (load $0.9 \times 10^6$ IFU), of the pure Namalva interferon proteins (A), and of the eluate from the Blue Dextran column (B). By comparison with Figure 1, it will be noted that the bands of the pure Namalva interferon are essentially identical with the bands of pure human leukocyte inteferon applied in the same amount.

Establishment of hybridoma cells with activity directed against interferon.

3 female Balb/c mice, age two months, were immunized with human leukocyte interferon in the following way:

The first injection (40,000 IFU) was performed subcutaneously in the back of each mouse. The immunization was continued every week with subcutaneous injection of 70,000 IFU. The last injection was given intravenously the 9th week (mouse 1) and 10th week (mice 2 and 3), respectively.

The development of anti-interferon was determined on serum samples from the mice, using the interferon neutralization test. As a laboratory check of the interferon neutralization test system, an internal anti-interferon IgG preparation (raised by injecting rabbits with partially purified human leukocyte interferon preparations) was, as usually, included. The serum samples from the mice showed no anti-interferon activity the first six weeks. Thereafter, distinct anti-interferon activity was found:

TABLE I.
IFU-NU per ml

|  | 7th Week | 8th Week | 9th Week | 10th Week |
|---|---|---|---|---|
| mouse 1 | 160 | 160 | 120 | — |
| mouse 2 | 200 | 1280 | 2500 | 1200—2500 |
| mouse 3 | 80 | 40 | 40 | 5—10 |

The mice were killed by breaking their necks two to four days after the last injection, and their spleens were removed under sterile conditions. After homogenization of each spleen in PBS, the homogenized cell suspension was transferred to centrifuge tubes and centrifugated for 5 minutes at 170 g at 4°C. The cells were resuspended in PBS, and after a second centrifugation, they were resuspended in serum-free DMEM (about 0.5 ml per spleen). The total amount of cells was $10^8$ (mouse 1), $0.8 \times 10^8$ (mouse 2), and $0.8 \times 10^8$ (mouse 3). The viability was around 85—90%.

9

By treatment with polyethylene glycol in the manner described below, the spleen cell suspension from each mouse was fused with $10^7 \times 63Ag8$ (HPRT-) myeloma cells in the following manner: $10^8$ mouse spleen cells and $10^7$ 8-azaguanin-resistent myeloma cells ($\times 63Ag8$; NSI/1Ag 4-1; SP 2/0-Ag 14) were mixed in a 50 ml conical plastic centrifuge tube (Falcon 2070). The tube was filled up with serum-free DMEM and centrifugated for 10 minutes at 170—200 g and 4°C. The supernatant was carefully removed, and at 37°C, a total of 0.7 ml of 50% polyethylene glycol solution having a temperature of 37°C was added dropwise over a period of 1 minute with gentle rotation. After incubation for 90 seconds at 37°C, 15 ml of warm serum-free DMEM were added very slowly (in the course of 1—2 minutes). Thereafter, the mixture was centrifugated for 10 minutes at 200 g, and the cell pellet was resuspended in 50 ml complete DMEM-FCS for seeding in Costar trays.

From each of the fusions, 48 cultures, each of 1 ml, were seeded in Costar trays (2 trays, each with 24 holes per spleen=48 cultures per mouse). After 10—15 days, growth was noted in 21 cultures (mouse 1), 0 cultures (mouse 2) and (after further seeding out) 150 cultures (mouse 3).

The cells were transferred to 5 ml cultures in 25 ml NUNC bottles which, like the Costar trays, contained a "feeder layer" of macrophages. On shift of medium, the supernatants were obtained, and from these dense cultures, cells were frozen in liquid nitrogen.

The supernatants of the individual cultures from mouse 1 were subjected to detection of positive clones using the interferon neutralization test. In this manner, one positive clone was found, although with a very low titer (about 2—3 IFU-NU per ml).

Production of anti-interferon by means of pure interferon proteins (pure by SDS PAGE).

The eluate from the above-described tandem affinity chromatography, as characterized by SDS PAGE, was used for immunization of rabbits as follows:

About 1,000,000 IFU units were concentrated to about 1 ml and dialyzed against PBS at 10°C overnight. Two rabbits were injected subcutaneously with each 1,000,000 IFU prepared in this manner. The injection was repeated each second week. The development of antibodies appears from Table II:

### TABLE II:
### Neutralization units (IFU-NU)

Freund's adjuvant

| Rabbit I xx) | 1 | 3 | 5 | 7 | 9 | 11 | 13 | 15 |
|---|---|---|---|---|---|---|---|---|
| xxx) | 0 | 0 | 2 | 100 | 2000 | ND[x)] | 20,000 | 20,000 |

Freund's adjuvant

| Rabbit I xx) | 17 | 19 | 21 | 23 | 25 | 27 |
|---|---|---|---|---|---|---|
| xxx) | 20,000 | 800,000 | 600,000 | 500,000 | 600,000 | 600,000 |

Freund's adjuvant

| Rabbit II xx) | 1 | 3 | 5 | 7 | 9 | 11 | 13 | 15 |
|---|---|---|---|---|---|---|---|---|
| xxx) | 0 | 0 | 0 | 20 | 500 | ND[x)] | 20,000 | 10,000 |

Freund's adjuvant

| Rabbit II xx) | 17 | 19 | 21 | 23 | 25 | 27 |
|---|---|---|---|---|---|---|
| xxx) | 8000 | 100,000 | 150,000 | 200,000 | 180,000 | 185,000 |

x) not determined
xx) week
xxx) antiinterferon titers (IFU-NU/ml)

Production of anti-interferon by means of pure stained interferon proteins cut out from an SDS PAGE.

The immunization was performed according to the protocol explained on page 11 of the present specification, using the minced interferon-containing (and partially washed and destained) gel suspension directly as the immunogenic preparation. One rabbit (III) was immunized with the 18,400±200 Daltons species, and another rabbit (IV) was immunized with the 20,100±200 Daltons species (died after week 15). Good results were obtained, vide Table III:

TABLE III:
Neutralization units

|  |  |  |  |  |  |  | Freund's adjuvant |  |
| --- | --- | --- | --- | --- | --- | --- | --- | --- |
|  |  |  |  |  |  |  | ↓ |  |
| Rabbit III (18,400 Daltons species) | week | 1 | 3 | 5 | 7 | 9 | 11 | 15 |
|  | IFU-NU/ml | 0 | 0 | 0 | 1—2 | 2 | 2 | 200 |
|  |  |  |  |  |  |  | ↓ |  |
| Rabbit IV (20,100 Daltons species) | week | 1 | 3 | 5 | 7 | 9 | 11 | 15 |
|  | IFU-NU/ml | 0 | 0 | 0 | 0 | 1 | 2 | 200 |

Antigenicity of the 18,400 Daltons species versus the 20,100 Daltons species and vice versa.

In order to show that the antigenic determinants of the above-mentioned two species are identical, the following cross-neutralization experiments were performed:

Interferon protein was eluted from the 18,400±200 Daltons species SDS PAGE band and the 20,100±200 Daltons species SDS PAGE band in the manner described above, and solutions containing 5—10 IFU of the two species were prepared. Solutions of anti-interferon from the two species, prepared in rabbits as described above, were diluted to contain 20 IFU-NU in total/ml. Aliquots (1 ml) of pure interferon species containing 10 IFU of the 18,400 Daltons interferon species and 10 IFU of the 20,100 Daltons interferon species, respectively, were mixed with 1 ml solution of the 18,400 Daltons species anti-interferon and 1 ml solution of the 20,100 Daltons species anti-interferon, respectively, in all possible permutations, that is, the anti-interferon of each species was mixed with the interferon of both species separately. After 1 hour at 37°C, any remaining interferon activity was determined by performing the usual interferon titration (vide "Materials and Methods" above). No interferon activity was found. Thus, when mixing the 18,400±200 Daltons species and the 20,100±200 Daltons species, respectively, with each of the anti-18,400±200 Daltons species and the anti-20,100±200 Daltons species, separately, and *vice versa*, no interferon was detected, in other words, a complete neutralization had occurred. Therefore, it can be concluded that the two interferon species exhibit the same antigenic determinants. This implies that the anti-18,400±200 Daltons species will be useable as a monospecific antibody for purification of both interferon species, and the same applies for the anti-20,100±200 Daltons species, and for a mixture of the two species. Further experiments performed in the same manner showed that each of the six biological peaks was completely neutralized by each of the antisera raised against the two major species.

It is highly likely that the two major species isolated from the Namalva SDS PAGE will give the same result, in other words, that they also cross-react and show identity to HuLeIf in terms of anti-genicity. (HuLeIF 18,400±200 being identical to Namalva 18,400±200, both with respect to antigenicity and molecular weight, and HuLeIF 20,100+200 being similarly identical with Namalva 20,100±200).

Biological effects of the pure interferon proteins.
Antiviral activity.

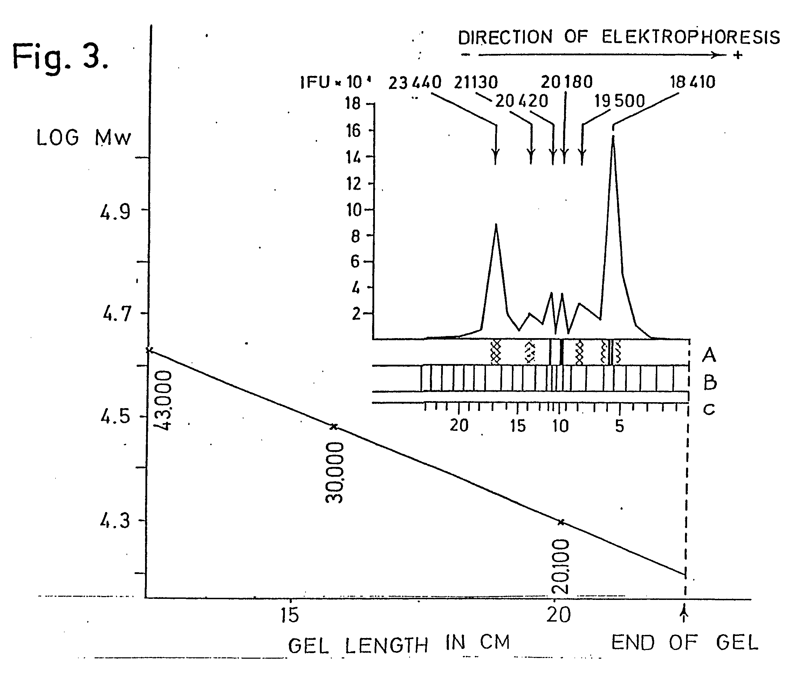

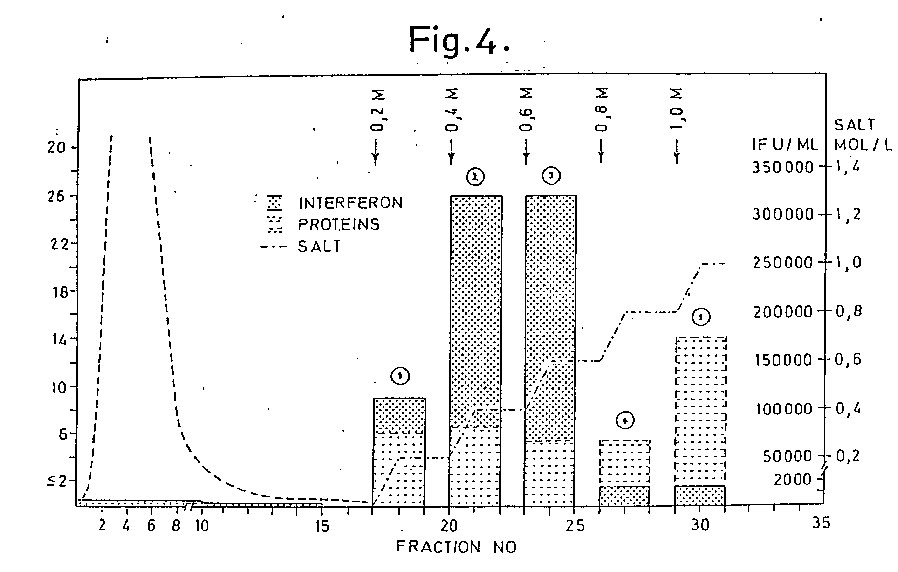

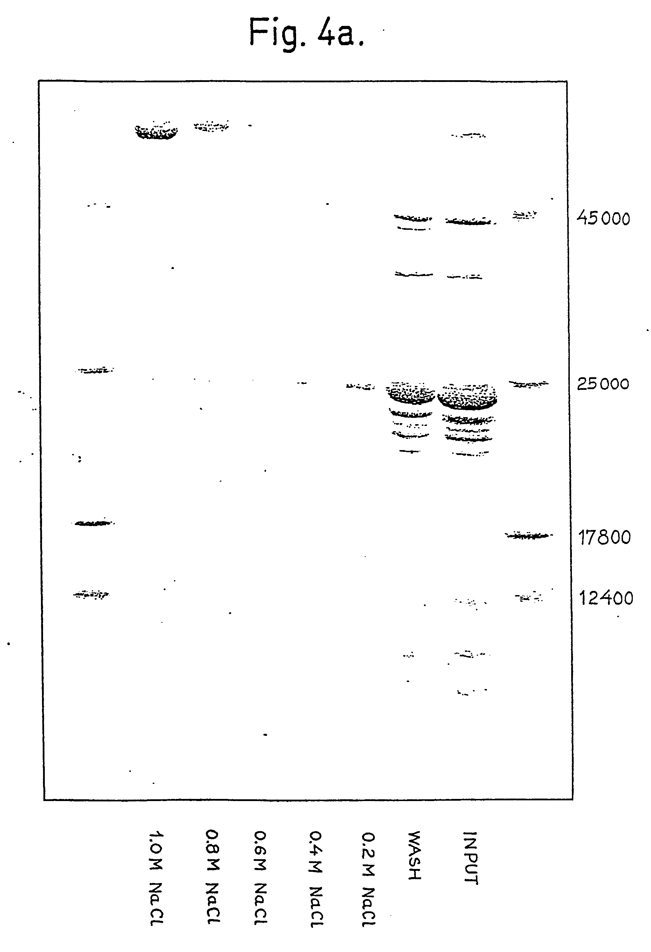

The antiviral activity of each of the six stained protein bands shown in Figure 3 was determined. The gel was loaded in two slots, both of which were stained. The stained bands in one of the slots is shown at A in Figure 3. The other slot slot was then briefly destained (in 50% methanol, 45% $H_2O_2$, 5% acetic acid), the exact location of the interferon proteins in the wet gel was recorded, and the gel was rinsed in water and was thereafter sliced as shown at B in Figure 3. The number of gel slices is indicated at C in Figure 3. In this manner, each interferon protein band was exactly cut out of the gel, without being mixed with the adjacent one. Each slice was eluted in the same manner as described in the section "Materials and Methods", and the biological profile shown in Figure 3 was constructed using the usual interferon titration described in "Material and Methods". The neutralizing activity of each of the six species cut out and eluted from the SDS PAGE was checked against anti-leukocyte interferon, and it was found that all of the species were completely neutralized by the same anti-serum. The recovery of interferon in Figure 3 was rather low (20%) compared with normal "SDS PAGE elution" without pre-staining (except for the 18,400±200 Daltons species), which indicates that the biological activity of most of the interferon species was selectively destroyed compared with the antigenicity. In the neutralization tests against anti-leukocyte interferon, the interferon proteins "eluted from Figure 3" were able to neutralize the anti-leukocyte interferon 3—5 times more effectively than native (crude) human leukocyte interferon, calculated on interferon activity basis, which indicates a selective destruction of determinants responsible for the biological activity.

Non-antiviral effects.

The non-antiviral effects of the pure human leukocyte interferon species were checked in 3 systems:

1) Anti-cellular activity.

The anticellular activity of the pure interferon proteins was investigated by incubating Daudi cells with 1:1000 dilutions (in medium) of pure interferon proteins obtained from the eluted SDS PAGE fractions as shown in Figure 2, by ascertaining the relative depression of Tritium labelled Thymidine (I. Heron and K. Berg, The actions of interferon are potentiated at elevated temperature, Nature, *274*, 508—510 (1978)) compared to controls without interferon (Figure 2, upper part, where "% G-I" designates % growth inhibition). As can be clearly seen, the "anticellular curve" follows the antiviral curve very strictly. This proves that all the five species of pure native human leukocyte interferon contain both the antiviral activity and the anticellular activity. The peak size of the different "anticellular peaks" does not vary linearly with the corresponding size of the "interferon peaks", which probably reflects the sensitivity of the Daudi cell system (J. Hilfenhaus, H. Damm, H. E. Karges and K. E. Manthey, Growth inhibition of human lymphoblastoid Daudi cells in vitro by interferon preparations, Arch. Virol. *51*, 87—97 (1976). The small interferon peak at 19,500 Daltons gave no rise to a corresponding peak in the anticellular curve. At a 10-fold lower dilution (1:100), however, a small but distinct peak of anticellular activity was also observed (not shown).

2) The expression of major histocompatibility antigen (MHC) on lymphocytes and monocytes.

The selective increase in $\beta_2$-associated MHC (major histocompatibility antigen) expression was observed using partially purified human leukocyte interferon, such as described by I. Heron, M. Hokland & K. Berg (1978), "Enhanced expression of $\beta_2$ microglobulin and HLA on human lymphoid cells by interferon", Proc. Natl. Acad. Sci. *75*: 6215—6222 (referred to below as PNAS *75*). Each of the two major human leukocyte interferon species (18,400 and 20,100 Daltons, vide Figure 1), was assayed in doses around 100 IFU per ml culture medium. The above-mentioned effect was found using these pure molecular species, whereas eluates from gel slices outside the regions where antiviral activity was recorded had no effect. It has, thus, been proved that the effect of selective enhancement of MHC antigen expression on lymphoid cells is an inherent feature of the interferon molecules.

3) The potentiation of the natural killer cell system (NK system).

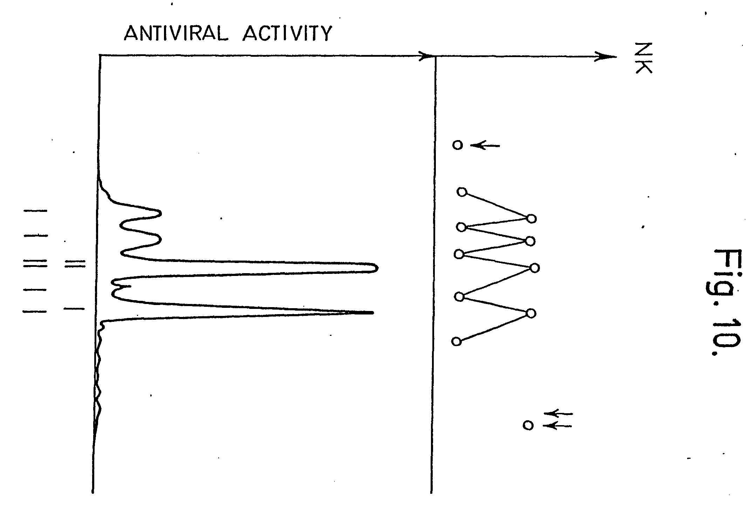

Figure 10 shows the antiviral profile (as assessed on an SDS PAGE in the same manner as described in connection with Figure 2). Each of the species from the gel was assessed for NK enhancing activity, using the method described in PNAS *75*. Fractions that have anti-viral activity as shown in the lower curve gave increased NK, such as illustrated in the upper curve, whereas "base line" fractions did not. One arrow indicates only saline added as a negative control, and two arrows indicate partially purified human leukocyte interferon (PIF) used as a positive control. Around 100 IFU antiviral units of each interferon preparation was added per ml.

## Claims

1. Antibodies raised or directed substantially only against, immunological determinants of human Le form interferon proteins which, under the SDS-PAGE and staining conditions defined herein, at a total interferon load of $0.9 \times 10^6$ IFU, show two major sharp stained protein bands having antiviral interferon activity at 18,400 and 20,100 Daltons, respectively, and a minor stained protein band having antiviral interferon activity between 20,300 and 20,400 Daltons, together with smaller peaks of antiviral interferon activity at 19,500, 21,130 and 23,440 Daltons (said Dalton molecular weights being subject to an experimental accuracy of ±200 Daltons), the stained protein regions of said SDS-PAGE acrylamide gradient being essentially only stained interferon proteins.

2. Antibodies as claimed in Claim 2, further characterised in that they are raised or directed substantially only against immunological determinants of human Le form interferon proteins which, under the SDS-PAGE and staining conditions defined herein, at a total interferon load of $3.8 \times 10^6$ IFU, show six stained protein bands having antiviral interferon activity, viz strong bands at 18,410 Daltons and 20,180 Daltons, respectively, a medium strong band at 20,400 Daltons and just visible bands at 19,500 Daltons, 21,130 Daltons, and 23,400 Daltons respectively (said Dalton molecular weights being subject to an experimental accuracy of ±200 Daltons), the peaks of antiviral interferon activity coinciding exactly with the stained protein bands, the stained protein regions of said SDS-PAGE acrylamide gradient being essentially only stained interferon proteins.

3. Antibodies raised or directed substantially only against immunological determinants of any one or a mixture of the individual proteins having antiviral interferon activity which are components of the human Le form interferon proteins referred to in Claim 1 or Claim 2.

4. Antibodies as claimed in Claim 3 obtained by immunising an immunisable animal with the 18,400±200, the 20,100±200 or a combination of the 18,400±200 and 20,100±200 Daltons human Le form interferon component of the proteins referred to in Claim 1 or Claim 2.

5. A method of preparing antibodies as claimed in any of Claims 1—4 comprising immunising an immunisable animal with the human Le form interferon proteins referred to in any of Claims 1—4, and obtaining antiserum from the animal.

6. A method as claimed in Claim 5 wherein the human Le form interferon proteins used for the immunisation are obtained from the respective band or bands cut from the SDS-PAGE gel.

7. A method as claimed in Claim 6 wherein the bands were cut from the SDS-PAGE gel after staining of said gel and a short wash in distilled water.

8. A method as claimed in any of Claims 5—7 wherein the immunisation is performed with a stabilized aqueous solution of the interferon protein(s) wherein the stabilizer present in the stabilized aqueous solution is SDS.

9. A method as claimed in Claim 8 wherein the stabilized aqueous solution of the interferon protein(s) is buffered with PBS at a pH of about 7.2.

10. A method as claimed in Claim 8 or Claim 9 wherein the stabilized aqueous solution contains an adjuvant.

11. A method as claimed in Claim 8 wherein the adjuvant is Freund's adjuvant.

12. A method for producing antibodies as claimed in any of Claims 1—4 comprising culturing a hybridoma cell clone, derived from the myeloma cell line X63 Ag 8; NSI/1Ag 4-1; SP 2/0-Ag 14, producing antibodies directed against immunological determinants of human Le form interferon proteins as referred to in any of Claims 1—4, and recovering antibodies from the culturing medium.

13. Antibodies when prepared by a method claimed in any of Claims 5—12.

14. Antibodies as claimed in any of Claims 1—4 or 13 immobilised on a matrix.

15. Antibodies as claimed in Claim 14 covalently bound to the matrix.

16. Antibodies as claimed in Claim 15 wherein the matrix is a cross-linked agarose such as Sepharose® 4B.

17. Matrix-immobilised antibodies as claimed in any of Claims 14—16 which have been treated to reduce proteolytic enzymatic activity.

18. Matrix-immobilised antibodies as claimed in Claim 17 which have been treated with enzyme inhibitors or enzyme destructurs.

19. Matrix-immobilised antibodies as claimed in Claim 18 for which the treatment with enzyme inhibitors or enzyme destructors has been carried out with matrix-immobilised enzyme inhibitor or enzyme destructor.

20. Matrix-immobilised antibodies as claimed in Claim 19 which, prior to covalent binding to the matrix, have been passed through a column of matrix-immobilised poly-L-lysin and/or matrix-immobilised Soyabean Trypsin inhibitor, and/or matrix-immobilised Kallikrein inactivator.

21. The use of antibodies as claimed in any of the claims 1—4 and 13—20 for purifying human Le form interferon-containing solutions.

**Patentansprüche**

1. Antikörper, die im wesentlichen nur gegen immunologische Determinanten von menschlichen Le - Form - Interferonproteinen gezüchtet oder gerichtet sind, die unter den hier definierten SDS PAGE- und Färbe - Bedingungen bei einer Gesamt - Interferonmenge von $0,9×10^6$ IFU zwei deutlich gefärbte Proteinhauptbanden von 18 400 bzw. 20 100 Dalton mit antiviraler Interferonaktivität und eine gefärbte Proteinnebenbande von 20 300 bis 20 400 Dalton mit antiviraler Interferonaktivität und kleinere Peaks von 19 500, 21 130 und 23 440 Dalton mit antiviraler Interferonaktivität aufweisen (wobei die experimentelle Genauigkeit dieser Molekulargewicht in Dalton±200 Dalton ist), wobei die gefärbten Proteinbereiche des SDS-PAGE Acrylamid - Gradienten im wesentlichen nur gefärbte Interferonproteine darstellen.

2. Antikörper nach Anspruch 1, die weiter dadurch gekennzeichnet sind, daß sie im wesentlichen nur gegen immunologische Determinanten von menschlichen Le - Form - Interferonproteinen gezüchtet oder gerichtet sind, die unter den hier definierten SDS-PAGE- und Färbe - Bedingungen bei einer Gesamt - Interferonmenge von $3,8×10^6$ IFU sechs gefärbte Proteinbanden mit antiviraler Interferonaktivität aufweisen, nämlich starke Banden von 18 410 Dalton bzw. 20 180 Dalton, eine mittelstarke Bande von 20 400 Dalton und gerade noch sichtbare Banden von 19 500 Dalton, 21 130 Dalton bzw. 23 400 Dalton (wobei die experimentelle Genauigkeit dieser Molekulargewichte in Dalton±200 Dalton ist), wobei die Peaks der antiviralen Interferonaktivität genau mit den gefärbten Proteinbanden übereinstimmen und die gefärbten Proteinbereiche des SDS-PAGE Acrylamid - Gradienten im wesentlichen nur gefärbte Interferonproteine darstellen.

3. Antikörper, die im wesentlichen nur gegen immunologische Determinanten eines einzelnen Proteins oder eines Gemisches von Proteinen mit antiviraler Interferonaktivität gezüchtet oder gerichtet sind, die Komponenten der in Anspruch 1 oder Anspruch 2 genannten menschlichen Le - Form - Interferonproteine sind.

4. Antikörper nach Anspruch 3, erhalten durch Immunisierung eines immunisierbaren Tieres mit der 18 400±200 oder der 20 100±200 Dalton großen menschlichen Le - Form - Interferonproteinkomponente der in Anspruch 1 oder Anspruch 2 beschriebenen Proteine oder mit einer Kombination dieser 18 400±200 und 20 100±200 Komponenten.

5. Verfahren zur Herstellung von Antikörpern nach einem der Ansprüche 1 bis 4, wobei man ein

# EP 0 091 543 B1

immunisierbares Tier mit einem der in den Ansprüchen 1 bis 4 beschriebenen Le - Form - Interferonproteine immunisiert und das Antiserum aus diesem Tier gewinnt.

6. Verfahren nach Anspruch 5, wobei die zur Immunisierung verwendeten menschlichen Le - Form - Interferonproteine aus der oder den entsprechenden Bande oder Banden, die aus dem SDS-PAGE-Gel herausgeschnitten wurden, erhalten werden.

7. Verfahren nach Anspruch 6, wobei die Banden aus dem SDS-PAGE-Gel ausgeschnitten wurden, nachdem das Gel gefärbt und kurz mit destilliertem Wasser gespült wurde.

8. Verfahren nach einem der Ansprüche 5 bis 7, wobei die Immunisierung mit einer stabilisierten wäßrigen Lösung des Interferonproteins (der Interferonproteine) durchgeführt wird, wobei der Stabilisator in der stabilisierten wäßrigen Lösung SDS ist.

9. Verfahren nach Anspruch 8, wobei die stabilisierte wäßrige Lösung des Interferonproteins (der Interferonproteine) durch PBS bei einem pH-Wert von etwa 7,2 gepuffert ist.

10. Verfahren nach Anspruch 8 oder Anspruch 9, wobei die stabilisierte wäßrige Lösung ein Adjuvans enthält.

11. Verfahren nach Anspruch 8, wobei das Adjuvans Freund's Adjuvans ist.

12. Verfahren zur Herstellung von Antikörpern nach einem der Ansprüche 1 bis 4, wobei man einen von der Myeloma-Zellinie X63 Ag 8; NSI/1Ag 4-1; SP 2/0-Ag 14 abstammenden Hybridoma - Zellclon züchtet, Antikörper erzeugt, die gegen immunologische Determinanten von in einem der Ansprüche 1 bis 4 beschriebenen menschlichen Le - Form - Interferonproteinen gerichtet sind, und die Antikörper aus dem Kulturmedium isoliert.

13. Antikörper, wenn sie nach einem der Verfahren der Ansprüche 5 bis 12 hergestellt wurden.

14. Antikörper nach einem der Ansprüche 1 bis 4 oder 13, die an einer Matrix immobilisiert sind.

15. Antikörper nach Anspruch 14, die kovalent an die Matrix gebunden sind.

16. Antikörper nach Anspruch 15, wobei die Matrix eine vernetzte Agarose wie Sepharose® 4B ist.

17. Matrix-immobilisierte Antikörper nach einem der Ansprüche 14 bis 16, die behandelt wurden, um die proteolytische enzymatische Aktivität zu vermindern.

18. Matrix-immobilisierte Antikörper nach Anspruch 17, die mit Enzyminhibitoren oder -abbauern behandelt worden sind.

19. Matrix-immobilisierte Antikörper nach Anspruch 18, wobei die Behandlung mit Enzyminhibitoren oder -abbauern mit Matrix - immobilisierten Enzyminhibitoren oder -abbauern durchgeführt worden ist.

20. Matrix-immobilisierte Antikörper nach Anspruch 19, die bevor sie kovalent an die Matrix gebunden werden, durch eine Säule mit Matrix - immobilisiertem Poly-L-Lysin und/oder Matrix - immobilisiertem Sojabohnen - Trypsin - Inhibitor und/oder Matrix - immobilisiertem Kallikrein - Inaktivator geleitet werden.

21. Verwendung der Antikörper nach einem der Ansprüche 1 bis 4 und 13 bis 20 zur Reinigung von menschliches Le - Form - Interferon - enthaltenden Lösungen.

**Revendications**

1. Anticorps élevés ou dirigés sensiblement uniquement contre les déterminants immunologiques des protéines d'interféron sous forme Le d'être humain qui, en conditions de SDS-PAGE et coloration définies ici, à une charge totale de l'interféron de $0,9 \times 10^6$ IFU, présentent deux bandes fortement teintées majeures de protéine ayant une activité antivirale de l'interféron à 18.400 et 20.100 Daltons, respectivement, et une bande de protéine, teintée mineure ayant une activité antivirale de l'interféron entre 20.300 et 20.400 Daltons, avec des petits pics d'activité antivirale de l'interféron à 19.500, 21.130 et 23.440 Daltons (lesdits poids moléculaires en Daltons étant soumis à une précision expérimentale de ±200 Daltons), les régions teintées de la protéine dudit gradient d'acrylamide SDS-PAGE étant essentiellement uniquement des protéines teintées de l'interféron.

2. Anticorps selon la revendication 1, caractérisés de plus en ce qu'ils sont élevés ou dirigés sensiblement uniquement contre les déterminants immunologiques des protéines d'interféron sous forme Le d'être humain qui, aux conditions de SDS-PAGE et de coloration définies ici, à une charge totale de l'interféron de $3,8 \times 10^6$ IFU, présentent six bandes teintées de protéine ayant une activité antivirale de l'interféron, c'est-à-dire des bandes fortes à 18.410 Daltons et 20.180 Daltons, respectivement, une bande moyennement forte à 20.400 Daltons et des bandes juste visibles à 19.500 Daltons, 21.130 Daltons et 23.400 Daltons, respectivement (lesdits poids moléculaires en Daltons étant soumis à une précision expérimentale de ±200 Daltons), les pics de l'activité antivirale de l'interféron coïncidant exactement avec les bandes teintées de la protéine, les régions teintées de la protéine dudit gradient acrylamide SDS-PAGE étant essentiellement uniquement des protéines teintées de l'interféron.

3. Anticorps élevés ou dirigés sensiblement uniquement contre les déterminants immunologiques de chacune ou d'un mélange de protéines individuelles ayant l'activité antivirale de l'interféron qui sont des composants des protéines de l'interféron sous forme de Le d'être humain indiquées à la revendication 1 ou la revendication 2.

4. Anticorps selon la revendication 3 obtenus par immunisation d'un animal immunisable avec le composant d'interféron sous forme Le d'être humain de 18.400±200, de 20.100±200 ou une combinaison de 18.400±200 et 20.100±200 Daltons des protéines indiquées à la revendication 1 ou à la revendication 2.

14

5. Méthode de préparation d'anticorps selon l'une quelconque des revendications 1—4 consistant à immuniser un animal immunisable par les protéines d'interféron sous forme Le d'être humain indiquées à l'une des revendication 1—4 et à obtenir l'antisérum de l'animal.

6. Méthode selon la revendication 5 où les protéines d'interféron sous forme Le d'être humain utilisées pour l'immunisation sont obtenues de la bande ou des bandes respectives découpées du gel SDS-PAGE.

7. Méthode selon la revendication 6 où les bandes ont été découpées du gel SDS-PAGE après coloration dudit gel et un court lavage à l'eau distillée.

8. Méthode selon l'une des revendications 5—7 où l'immunisation est accomplie avec une solution aqueuse stabilisée de la ou des protéines d'interféron où le stabilisant présent dans la solution aqueuse stabilisée est SDS.

9. Méthode selon la revendication 8 où la solution aqueuse stabilisée de la ou des protéines de l'interféron est tamponnée avec PBS à un pH d'environ 7,2.

10. Méthode selon la revendication 8 ou la revendication 9 où la solution aqueuse stabilisée contient un adjuvant.

11. Méthode selon la revendication 8 où l'adjuvant est l'adjuvant de Freund.

12. Méthode de production d'anticorps selon l'une des revendications 1—4 comprenant la mise en culture d'un clone de cellule d'hybridome, dérivé de la lignée de cellules de myéloma X63 Ag8; NSI/1Ag 4-1; SP 2/0-Ag 14, produisant des anticorps dirigés contre les déterminants immunologiques des protéines d'interféron sous forme Le d'être humain selon l'une des revendications 1—4, et de récupération des anticorps du milieu de culture.

13. Anticorps lorsqu'ils sont préparés par une méthode selon l'une quelconque des revendications 5—12.

14. Anticorps selon l'une quelconque des revendications 1—4 ou 13 immobilisés sur une matrice.

15. Anticorps selon la revendication 14 liés de manière covalente à la matrice.

16. Anticorps selon la revendication 15 où la matrice est un agarose réticulé tel que Sepharose® 4B.

17. Anticorps immobilisés sur matrice selon l'une des revendications 14—16 qui ont été traités pour réduire l'activité enzymatique protéolytique.

18. Anticorps immobilisés sur matrice selon la revendication 17 qui ont été traités avec des inhibiteurs d'enzyme ou des destructeurs d'enzyme.

19. Anticorps immobilisés sur matrice selon la revendication 18 pour lesquels le traitement avec les inhibiteurs d'enzyme ou les destructeurs d'enzyme a été effectué avec l'inhibiteur d'enzyme ou le destructeur d'enzyme immobilisé sur matrice.

20. Anticorps immobilisés sur matrice selon la revendication 19 qui, avant liaison covalente à la matrice, ont passé à travers une colonne de poly-L-lysine immobilisée sur matrice et/ou d'inhibiteur de trypsine du soja immobilisé sur matrice et/ou d'inactivateur de kallikréine immobilisé sur matrice.

21. Utilisation des anticorps selon l'une quelconque des revendications 1—4 et 13—20 pour la purification de solutions contenant un interféron sous forme Le d'être humain.

# Fig. 1.

20 300

20 100

18 400

Fig. 2.

Fig. 3.

DIRECTION OF ELEKTROPHORESIS

LOG Mw

# Fig.4.

EP 0 091 543 B1

EP 0 091 543 B1

Fig. 4a.

45000

25000

17800

12400

1.0 M NaCl

0.8 M NaCl

0.6 M NaCl

0.4 M NaCl

0.2 M NaCl

WASH

INPUT

5

Fig. 5.

EP 0 091 543 B1

Fig. 6.

EP 0 091 543 B1

# Fig. 7.

# Fig. 8.

# Fig. 9.

67 000

43 000

30 000

20 100

14 400

B      A

# Fig. 10.